(19) Europäisches Patentamt
European Patent Office
Office européen des brevets

(11) **EP 2 252 688 B1**

(12) **EUROPEAN PATENT SPECIFICATION**

(45) Date of publication and mention
of the grant of the patent:
**16.08.2017 Bulletin 2017/33**

(51) Int Cl.:
*C12N 15/113* (2010.01)   *A61K 31/7088* (2006.01)

(21) Application number: **09721382.1**

(86) International application number:
**PCT/EP2009/053002**

(22) Date of filing: **13.03.2009**

(87) International publication number:
**WO 2009/115465 (24.09.2009 Gazette 2009/39)**

(54) **SYNTHETIC PHOSPHODIESTER OLIGONUCLEOTIDES AND THERAPEUTICAL USES THEREOF**

Synthetische Phosphodiester-Oligonukleotide und deren therapeutische Verwendungen

Oligonucléotides phosphodiesters synthétiques et leurs utilisations thérapeutiques

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **19.03.2008 EP 08425175**
**07.05.2008 US 51088 P**

(43) Date of publication of application:
**24.11.2010 Bulletin 2010/47**

(73) Proprietor: **Gentium S.r.l.**
**22079 Villa Guardia (Como) (IT)**

(72) Inventors:
• **STEIN, Aaron Cy**
**New City**
**New York NY-10956 (US)**
• **IACOBELLI, Massimo**
**I-20152 Milano (IT)**

(74) Representative: **Pistolesi, Roberto et al**
**Dragotti & Associati Srl**
**Via Nino Bixio, 7**
**20129 Milano (IT)**

(56) References cited:
WO-A-01/78761   WO-A-87/06235
WO-A-2005/023273   WO-A-2006/094916
WO-A-2006/119619

• **KORNBLUM, N. ET AL.: "Defibrotide, a polydisperse mixture of single-stranded phosphodiester oligonucleotides with lifesaving activity in severe hepatic veno-occlusive disease: Clinical outcomes and potential mechanisms of action." OLIGONUCLEOTIDES, vol. 16, no. 1, 2006, pages 105-114, XP002492851 ISSN: 1545-4576 cited in the application**
• **BENIMETSKAYA LUBA ET AL: "Angiogenesis alteration by defibrotide: implications for its mechanism of action in severe hepatic veno-occlusive disease" BLOOD, vol. 112, no. 10, November 2008 (2008-11), pages 4343-4352, XP008106635 ISSN: 0006-4971**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The invention relates to mixtures of synthetic phosphodiester oligonucleotides called Nmers ranging from 45 mers to 55 mers for use in the treatment and/or prevention of veno-occlusive disease. The phosphodiester oligonucleotides are preferably heteropolymers composed of either A, G, C, and T at each position but may also be homopolymers, *i.e.* the same base may be present at each position in the oligonucleotide.

**BACKGROUND OF THE INVENTION**

The term defibrotide identifies a complex mixture of single stranded oligonucleotides (15-80mer, average 45mer) obtained by extraction from animal and/or vegetable tissue and, in particular, from the intestines of a pig or cow (US 3,770,720 and US 3,899,481). Defibrotide, which has an average molecular weight of 16.5 $\pm$ 2.5 kDa, is normally used in the form of a salt of an alkali metal, generally sodium. It is principally used for its antithrombotic activity (US 3,829,567) although it may be used in different applications, such as, for example, the treatment of acute renal insufficiency (US 4,694,134) and the treatment of acute myocardial ischemia (US 4,693,995). Additional literature on defibrotide is cited below.

US 5,081,109 discloses the use of defibrotide to treat peripheral arteriopathies in advanced phase (phase III and IV).

US 5,116,617 discloses methods of strengthening capillaries in humans comprising topically applying compositions containing defibrotide.

US 5,977,083 discloses that various disease states can be treated by modifying the dose of defibrotide in response to observed fluctuations (e.g., increase, decrease, appearance, disappearance) in normal, disease and repair markers.

US 6,046,172 discloses oligodeoxyribonucleotides of animal origin, having a molecular weight comprised between 4000 and 10000 Daltons, which can be obtained by fractionation of polydeoxyribonucleotides or otherwise by chemical or enzymatic depolymerization of high molecular weight deoxyribonucleic acids.

US 6,699,985 and US 5,624,912 disclose a method of using defibrotide to treat various disease conditions, including HIV infection.

US 7,338,777 discloses a method of determining the biological activity of defibrotide.

EP1276497 discloses a method of increasing the amount of stem cells and progenitor cells in the peripheral blood of a mammal by the administering defibrotide in combination or in temporal proximity with at least one haematopoietic factor (such as G-CSF) having the capacity to mobilise haematopoietic progenitors.

WO2005023273 discloses the anti-tumor action of defibrotide.

WO2006094916 describes the use of defibrotide for treating angiogenesis-dependent tumors.

Additionally, a review article, "Defibrotide, a Polydisperse Mixture of Single Stranded Phosphodiester Oligonucleotides with Lifesaving Activity in Severe Hepatic Veno-occlusive Disease: Clinical Outcomes and Potential Mechanisms of Action," by Kornblum et al. (Oligonucleotides, 16:105-114 (2006)), discusses defibrotide and its use in treating veno-occlusive disease (VOD).

In 1998, Dr. Paul Richardson of the Dana-Farber Cancer Institute in Boston, Massachusetts began using defibrotide as a treatment for severe hepatic VOD after bone marrow transplantation. The drug was dosed intravenously with almost no toxicity, and it cured about 40%-50% of patients in a disease that was hitherto fatal in 95% of cases. Subsequent multi-institutional trials in the United States and Europe have confirmed the efficacy of defibrotide, although its mechanism of action is unknown.

US 4,985,552 and US 5,223,609 describe a process for the production of defibrotide which enables a product to be obtained which has constant and well defined physico-chemical characteristics and is also free from any undesired side-effects.

EP1325162 discloses a method for determining the biological activity of defibrotide.

For decades the dogma has been that phosphodiester oligonucleotides cannot be used as drugs because of nuclease digestion, but this attitude neglects the large quantities in which they can be administered to patients due to their low toxicity. Clinical experience with defibrotide clearly indicates that these types of molecules can be given with therapeutic efficacy. However, there is some question as to whether or not defibrotide can be reproduced identically, as it is a natural product. Therefore, there is a need in the art to develop a composition that has the same effect as defibrotide but that will be able to be identically reproduced.

## SUMMARY OF THE INVENTION

The present invention is directed to a pharmaceutical formulation which is an aqueous solution and which consists of: (a) water, (b) a mixture of synthetic phosphodiester oligonucleotides having a length of from 45 bases to 55 bases or from about 50 bases to about 55 bases, and optionally pharmaceutically acceptable excipients and/or adjuvants) and no other ingredient which materially affects the activity of the synthetic phosphodiester oligonucleotides, for use in the treatment and/or prevention of veno-occlusive disease. In the invention said oligonucleotides may be single stranded; the sequences of said oligonucleotides may DNA and/or RNA sequences; the sequences of said oligonucleotides may also be random sequences.

In the invention the purine bases of said oligonucleotides may be selected from guanine, adenine, xanthine and hypoxanthine and the pyrimidine bases may be selected from cytosine, thymine, methylcytosine and uracil; the sugar of said oligonucleotides may be selected from ribose and deoxyribose.

These and other aspects of the present invention will become apparent upon reference to the following detailed description and attached drawings.

## DETAILED DESCRIPTION

DEFINITIONS

The following definitions are given for a better understanding of the present invention:

A nucleotide is a chemical compound that consists of 3 portions: a heterocyclic base, a sugar, and one or more phosphate groups. In the most common nucleotides, the base is a derivative of purine or pyrimidine and the sugar is the pentose (five-carbon sugar) deoxyribose or ribose. Nucleotides are the monomers of nucleic acids such as DNA or RNA.

Oligonucleotides are short sequences of nucleotides, typically with twenty or fewer bases. Automated synthesizers allow the synthesis of oligonucleotides up to 160 to 200 bases. The length of a synthesized base is usually denoted by 'mer' (from 'Greek' meros "part"). For example, a fragment of 25 bases would be called a 25-mer.

A phosphodiester bond is a group of strong covalent bonds between the phosphorus atom in a phosphate group and two other molecules over two ester bonds. Phosphodiester bonds make up the backbone of the strands of DNA and RNA.

DNA and RNA are long polymers of simple units called nucleotides, with a backbone made of sugars and phosphate groups joined by phosphodiester bonds. Attached to each sugar is one of four types of molecules called bases. In DNA and RNA, the phosphodiester bond is the linkage between the 3' carbon atom and the 5' carbon of the ribose sugar.

DNA is often double stranded and normally contains two types of purine bases, guanine and adenine, and two types of pyrimidine bases, cytosine and thymine. In certain cases, purine and pyrimidine bases can be replaced by their mutated forms: guanine and adenine may be replaced by xanthine and hypoxantine, respectively, whereas cytosine may be replaced by methylcytosine. RNA is very similar to DNA, but differs in a few important structural details: RNA is typically single stranded, while DNA is typically double stranded. Also, RNA nucleotides contain ribose sugars while DNA contains deoxyribose; furthermore, RNA contains uracil instead of thymine which is present in DNA.

A random nucleotide sequence is a nucleotide sequence essentially containing an equal mixture of two different purine bases and two different pyrimidine bases wherein, at each position of the sequence, each purine or pyrimidine base has a 25% $\pm$ 5 probability of being present, preferably 25% $\pm$ 2, more preferably 25% $\pm$ 1.

As used herein, the term "isolated" means that the material being referred to has been removed from the environment in which it is naturally found, and is characterized to a sufficient degree to establish that it is present in a particular sample. Such characterization can be achieved by any standard technique, such as, e.g., sequencing, hybridization, immunoassay, functional assay, expression, size determination, or the like. Thus, a biological material can be "isolated" if it is free of cellular components, *i.e.*, components of the cells in which the material is found or produced in nature.

An isolated organelle, cell, or tissue is one that has been removed from the anatomical site (cell, tissue or organism) in which it is found in the source organism. An isolated material may or may not be "purified". The term "purified" as used herein refers to a material (*e.g.*, a nucleic acid molecule or a protein) that has been isolated under conditions that detectably reduce or eliminate the presence of other contaminating materials. Contaminants may or may not include native materials from which the purified material has been obtained. A purified material preferably contains less than about 90%, less than about 75%, less than about 50%, less than about 25%, less than about 10%, less than about 5%, or less than about 2% by weight of other components with which it was originally associated.

The practice of the present invention will employ, unless indicated specifically to the contrary, conventional methods of molecular biology, cell biology and protein chemistry within the skill of the art, many of which are described below for the purpose of illustration. Such techniques are explained fully in the literature. See, *e.g.*, Sambrook, et al., "Molecular Cloning: A Laboratory Manual" (2nd Edition, 1989); "DNA Cloning: A Practical Approach, vol. I & II" (D. Glover, ed.); "Oligonucleotide Synthesis" (N. Gait, ed., 1984); "Nucleic Acid Hybridization" (B. Hames & S. Higgins, eds., 1985); Perbal, "A Practical Guide to Molecular Cloning" (1984); Ausubel et al., "Current protocols in Molecular Biology" (New York, John Wiley and Sons, 1987); and Bonifacino et al., "Current Protocols in Cell Biology" (New York, John Wiley & Sons, 1999).

The term "about" means within an acceptable error range for the particular value as determined by one of ordinary skill in the art, which will depend in part on how the value is measured or determined, *i.e.*, the limitations of the measurement system. For example, "about" can mean within an acceptable standard deviation, per the practice in the art. Alternatively, "about" can mean a range of up to $\pm20\%$, preferably up to $\pm10\%$, more preferably up to $\pm5\%$, and more preferably still up to $\pm1\%$ of a given value. Alternatively, particularly with respect to biological systems or processes, the term can mean within an order of magnitude, preferably within 2-fold, of a value. Where particular values are described in the application and claims, unless otherwise stated, the term "about" is implicit and in this context means within an acceptable error range for the particular value.

In the context of the present invention insofar as it relates to any of the disease conditions recited herein, the terms "treat", "treatment", and the like mean to prevent or relieve or alleviate at least one symptom associated with such condition, or to slow or reverse the progression of such condition. For example, within the meaning of the present invention, the term "treat" also denotes to arrest, delay the onset (*i.e.*, the period prior to clinical manifestation of a disease) and/or reduce the risk of developing or worsening a disease. The term "protect" is used herein to mean prevent, delay or treat, or all, as appropriate, development or continuance or aggravation of a disease in a subject.

The phrase "pharmaceutically acceptable", as used in connection with compositions of the invention, refers to molecular entities and other ingredients of such compositions that are physiologically tolerable and do not typically produce untoward reactions when administered to an animal such as a mammal (*e.g.*, a human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

As used herein, the expression "mixture of synthetic phosphodiester oligonucleotides" means a mixture of synthetic phosphodiester oligonucleotides which may have the same and/or different sequences. According to a first embodiment, the mixture may include both oligonucleotides having the same sequence and oligonucleotides having different sequences; on their turn, such oligonucleotides having different sequences may have the same or different lenghts. According to a second embodiment, the mixture may consist of oligonucleotides having different sequences but the same lenght.

The terms "administering" or "administration" are intended to encompass all means for directly and indirectly delivering a compound to its intended site of action.

The term "animal" means any animal, including mammals and, in particular, humans.

## BRIEF DESCRIPTION OF THE DRAWINGS

The attached figures are included solely to illustrate the preferred embodiment of the present invention without limiting the invention in any manner whatsoever, wherein:

FIG. 1A is a band intensity showing competition by defibrotide and defibrotide molecular weight fractions for binding of ClRNH$^{32}$P-OdT$_{18}$ to bFGF.

FIG. 1B is a plot of the normalized band intensity versus the log of the defibrotide or defibrotide molecular weigh fractions concentrations.

FIG. 2 is a chart and table showing a comparison of $K_c$ values for defibrotide and defibrotide molecular weight fraction and Nmer competitors of ClRNH$^{32}$P-OdT$_{18}$ binding to bFGF.

FIG. 3A is a band intensity showing modification of PDGF BB by alkylating oligodeoxynucleotide, ClRNH$^{32}$P-OdT$_{18}$.

FIG. 3B is a plot of relative band intensity versus reactive oligodeoxynucleotide concentration.

FIG. 3C is a double reciprocal plot of the data in FIG. 3B.

FIG. 4 is a chart and table showing a comparison of the $K_c$ values for defibrotide, defibrotide molecular weight fraction and Nmer competitors of ClRNH$^{32}$P-OdT$_{18}$ binding to bFGF.

FIG. 5 is a chart and table showing a comparison of the $K_c$ values for Nmer and Tmer competitors of C1RNH$^{32}$P-OdT$_{18}$ binding to VEGF.

FIG. 6 is a chart and table showing a comparison of the $K_c$ values for Nmer and Tmer competitors of C1RNH$^{32}$P-OdT$_{18}$ binding to laminin.

FIG. 7 is a chart and table showing a comparison of the $K_c$ values of Nmer and Tmer competitors of C1RNH$^{32}$P-OdT$_{18}$ binding to laminin.

FIG. 8A is a chart of inhibition of bFGF-mediated HMEC-1 proliferation by defibrotide.

FIG. 8B is a chart of the inhibitory effects of defibrotide on cell growth in the absence of bFGF.

FIG. 9A is a chart of inhibition of bFGF-mediated HMEC-1 proliferation by Nmers.

FIG. 9B is a chart of the inhibitory effects of Nmers on cell growth in the absence of bFGF.

FIG. 10 is a chart showing the effect of defibrotide and Nmers on the partial thromboplastin time (PTT).

FIG. 11A is a chart showing the dose-dependent release of TFPI to conditioned medium by exposure of HMEC-1 cells to increasing concentrations of defibrotide for 24 hours.

FIG. 11B is a chart showing the time-course of the TFPI release to conditioned medium induced by 5 $\mu$M defibrotide.

FIG. 11C is a chart showing the dose-dependent release of TFPI to conditioned medium by exposure of HMEC-1 cells to increasing concentrations of defibrotide for 30 minutes.

FIG. 12A is a chart showing the dose-dependent release of TFPI into conditioned medium by exposure of HMEC-1 cells to increasing concentrations of defibrotide molecular weight fractions.

FIG. 12B is a chart showing the time-course of the TFPI release into conditioned medium induced by 5 $\mu$M defibrotide.

FIG. 13 is a chart showing the ability of defibrotide and Nmers to substitute for heparin in the bFGF + heparin-stimulated proliferation of FGFR2-transfected C11 cells.

## DETAILED DESCRIPTION OF THE INVENTION

Oligonucleotides, such as defibrotide, can bind to proteins that bind to heparin. As used herein, the term heparin means low-affinity heparin. Synthetic analogs of defibrotide can be made that have comparable or higher activity than the natural product, and these analogs have anti-cancer activity because of their ability to bind to heparin-binding growth factors. Three heparin-binding proteins of great importance to cancer cells include basic fibroblast growth factor (bFGF), vascular endothelial growth factor (VEGF) and laminin; the composition of the present invention can bind to these proteins with nanomolar affinity, yet this binding is not sequence-specific.

The present composition is based on the surprising finding that mixtures of synthetic phosphodiester oligonucleotides having a length of from about 40 mers to about 65 mers recapitulate the properties of defibrotide and may thus be used as a synthetic alternative to such an active principle. The oligonucleotides of the present invention have a length of about 45-55 mers, preferably of about 50-55 mers.

The purine bases of the oligonucleotides of the present invention are preferably selected from guanine, adenine, xanthine and hypoxanthine and the pyrimidine bases are selected from cytosine, thymine, methylcytosine and uracil. According to one embodiment, the sequences would be composed of a mixture of each genetic base (A, G, C, and T) at each position in the oligonucleotides; preferably, they would be random sequences. According to another embodiment the sequences would consist of the same base (such as thymidine, i.e. Tx) at each position in the oligonucleotides (known as the Tm series, or Tmers). According to a further embodiment, the sugar of the present oligonucleotides is selected from ribose and deoxyribose.

According to another embodiment, the oligonucleotides of the present invention consists of DNA and/or RNA sequences.

According to a preferred embodiment, the oligonucleotides of the present invention are single stranded.

As it will be apparent from the experimental section, the present inventors have surprisingly found that the fractions of defibrotide having low molecular weight and, in particular, those having a molecular weight lower than 40 kDa, are those having the lower ability to bind to heparin-binding growth factors. Such a finding has thus allowed for the selection of well-defined mixtures of oligonucleotides that can be easily and identically reproduced and that can mimic the effects of defibrotide.

The mixtures of the present invention can thus be used to treat mammalian patients, preferably human, afflicted with those diseases which would be treated by administering defibrotide, such as VOD, thrombotic thrombocytopenic purpura (TTP), tumors, angiogenesis dependent tumors (such as multiple myeloma or breast carcinoma); those mixtures might also be used as blood anticoagulant or for increasing the amount of stem cells and progenitor cells in the peripheral blood of a mammal when administered in combination or in temporal proximity with at least one hematopoietic factor having the capacity to mobilize hematopoietic progenitors.

The mixtures of oligonucleotides of the present invention may be administered in the same way as defibrotide; preferably, they would be administered by injection, preferably intravenously, by means of an aqueous solution. Such aqueous solution may have oligonucleotide concentrations from 5 to 60 micromoles/liter, preferably from 10 to 50 micromoles/liter.

## EXAMPLES

The present invention will be better understood by reference to the following non-limiting examples.

MATERIALS AND METHODS

Generation of synthetic phosphodiester oligonucleotides In order to create the series of Nmers, a DNA sequencing machine (commonly available on the market, by, for example ABI or Millipore) was used. Equal amounts (as measured by molarity) of each base (adenine, cytosine, guanine, and thymine) were used in the sequencing reaction. The machine was programmed to make random lengths of single-stranded DNA ranging in size from 25 bases to 200 bases, and each base was chosen at random from the four genetic bases.

*Cell culture*

SV40-transformed HMEC-1 cells were obtained from the CDC in Atlanta, GA. They were grown in MCDB 131 media supplemented with 10% heat inactivated fetal bovine serum (FBS), 10 ng/ml EGF, 1 $\mu$g/mL hydrocortisone, 100 U/mL penicillin G sodium and 100 $\mu$g/ml streptomycin sulfate. The mycoplasma-free human melanoma cell line 518A2 was obtained from Dr. Volker Wacheck of the University of Vienna in Austria. Cells were grown in DMEM supplemented with 10% heat inactivated FBS and 100 U/ml penicillin G sodium and 100 $\mu$g/ml streptomycin sulfate. The human hepatic stellate LX2 cell line was generated by SV40 T antigen spontaneous immortalization in low serum conditions, and was provided by Dr. Scott L. Friedman of the Mount Sinai School of Medicine in New York. LX2 cells were grown in DMEM supplemented with 1% heat inactivated FBS and 100 U/ml penicillin G sodium and 100 $\mu$g/ml streptomycin sulfate. The stock cultures were maintained at 37°C in a humidified 5 % $CO_2$ incubator.

*Generation of defibrotide, defibrotide molecular weight fractions, and synthetic phosphodiester o ligonucleo tides*

Defibrotide, a highly complex polydisperse material composed of single-stranded phosphodiester polydeoxyribonucleotides (molecular weight is 16.5 $\pm$ 2.25 kDa), was prepared via controlled depolymerization of DNA extracted from porcine intestinal tissue, and was provided by Gentium (Como, Italy). Defibrotide molecular weight fractions, which is defibrotide isolated from porcine intestinal tissue and then fractionated, (A2, E2, G2, I2, L2 with molecular weights 9,353; 12,258; 16,761; 21,840; and 26,190 Daltons, respectively) were also supplied by Gentium. Nmers (a series of synthetic phosphodiester oligonucleotides of various, defined lengths,) and Tmers (a series of phosphodiester homopolymers of thymidine of defined length) were synthesized, purified via the procedure detailed above and supplied by Trilink Biotechnologies (San Diego, CA).

*Recombinant proteins and cell culture materials*

Recombinant human bFGF and VEGF165, platelet-derived growth factor-BB (PDGF BB) and heparin-binding epidermal growth factor-like growth factor (HB-EGF) were purchased from R&D Systems (Minneapolis, MN). Laminin was obtained from Sigma-Aldrich (St. Louis, MO). DMEM, MCDB 131, M199 medium, and FBS were obtained from Invitrogen (Carlsbad, CA). Fibronectin-coated plates and Matrigel were purchased from BD Bioscience (Bedford, MA). The IMUBIND Total TFPI ELISA kit was obtained from American Diagnostica (Stanford, CT).

*Syntheses of the alkylating oligodeoxynucleotide probe CIRNH$^{32}$p-OdT$_{18}$*

Ten OD U of OdT$_{18}$ were 5'-labeled with [$^{32}$P]phosphate by reaction with 5'-polynucleotide kinase. Excess ATP was separated from the reaction product by Sephadex G25 chromatography in 0.1 M lithium perchlorate. The oligonucleotide was then precipitated by addition of 2% LiClO$_4$/acetone and dissolved in water at a concentration of 200 OD U/$\mu$l. The oligonucleotide was then precipitated by the addition of 8% aqueous solution of cetyltrimethylammonium bromide solution and dried. 6.5 mg of p-(benzylamino)-N-chloroethyl-N-methylamine (CIRNH$_2$) in 20$\mu$l of dimethylformamide, followed by 8 mg of dipyridyl disulfide and 9.5 mg of triphenylphosphine was then added to the dried oligonucleotide. After 2 hours, the oligonucleotide was precipitated by addition of 2% LiClO$_4$/acetone, dissolved in 25 $\mu$l of 1M NaCl, precipitated with ethanol and dried. The final product was redissolved in water, and stored at -80°C.

*Modification of heparin-binding proteins by CIRNH$^{32}$P-OdT$_{18}$*

Modification of heparin-binding proteins by C1RNH$^{32}$P-OdT$_{18}$ was accomplished by the method of Yakubov et al. (Oligonucleotides interact with recombinant CD4 at multiple sites. J. Biol. Chem. 1993 268:19918-18823). Initially, bFGF (50 nM concentration), PDGF BB (500 nM), VEGF (150 nM), laminin (50 nM) or HB-EGF (400 nM) was incubated in 0.1 M Tris-HCl, pH 7.4, containing 10-20 $\mu$M ClRNH$^{32}$P-OdT$_{18}$. Defibrotide, defibrotide molecular weight fractions, Tmers or Nmers were used at increasing concentrations as competitors of the binding of the probe phosphodiester oligonucleotide to the proteins. After 1 hour at 37°C, one volume of a buffer containing 10% glycerol, 4% 2-mercaptoethanol, 4% SDS and 0.2% bromophenol blue was added, and SDS-PAGE was performed. The gels were dried and exposed to Kodak X-ray film until bands were visualized. The film was developed, and band densities were quantitated by laser densitometry.

*Proliferation assay*

Confluent HMEC-1 cells were treated in their place for 24 hours in M199 medium containing 2.5% FBS, and then seeded (2x10$^4$ cells/well) in Fibronectin-coated 96-well plates (in M199 medium supplemented with 2.5% FBS). Subsequently, the medium was then replaced with fresh medium containing either 20 ng/mL bFGF alone, defibrotide or Nmers with or without bFGF. After 3 days treatment at 37°C, the cell growth was evaluated by sulforhodamine B staining. All experiments were carried out in quadruplicate.

*Determination of **Tissue Factor Pathway Inhibitor** (TFPI) release*

HMEC-1 cells were seeded in 24-well plates in M199 medium containing 2.5% FBS at a density of 10x10$^4$ cells/well. The cells were treated with either defibrotide or defibrotide molecular weight fractions, or Nmers for different time intervals. Then, the conditioned cell media was collected, centrifuged at 10,000g for 10 minutes to remove cell debris, and the concentration of Tissue Factor Pathway Inhibitor (TFPI) in the medium was measured using an ELISA assay as described by the manufacturer.

RESULTS

*Defibrotide molecular weight fractions and Nmers interact similarly with heparin binding proteins*

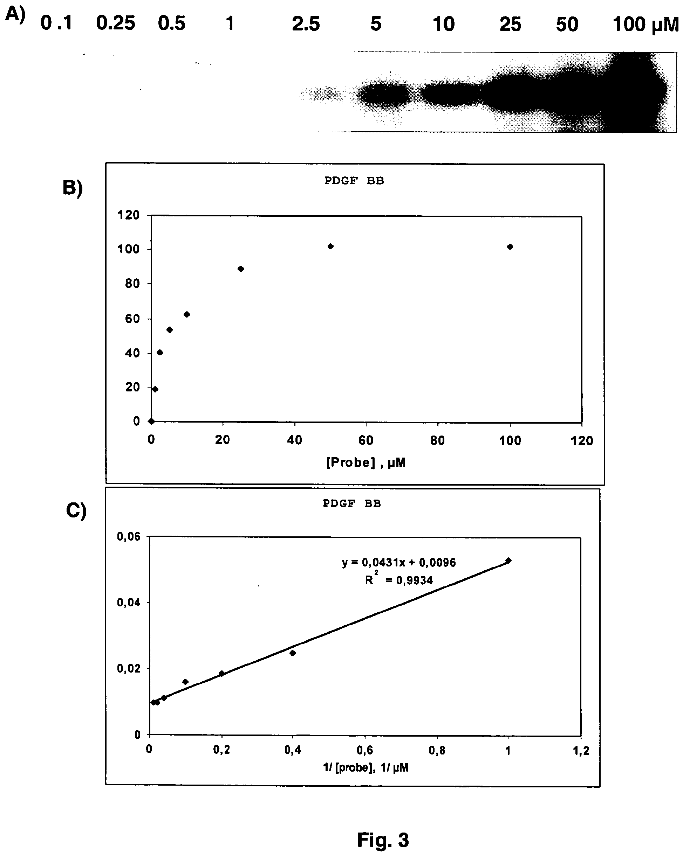

Defibrotide, defibrotide molecular weight fractions and Nmers interact with heparin-binding proteins that are important in tumor growth, viability, angiogenesis, and migration. The assessment of the ability of defibrotide, defibrotide molecular weight factions and Nmers to bind to heparin binding proteins was accomplished via a competition assay. In the first step, an alkylating, $^{32}$P-labeled phosphodiester 18mer homopolymer of thymidine (ClRNH$^{32}$P-OdT$_{18}$) was synthesized. This molecule was mixed with bFGF, PDGF BB, BB, VEGF, laminin or HB-EGF, incubated in 0.1 M Tris-HCl, pH 7.4, containing 10-20 $\mu$M of labeled probe and with increasing concentrations of defibrotide, defibrotide molecular weight fractions or Nmers. The mixture was then separated by gel electrophoresis and autoradiographed. Defibrotide, defibrotide molecular weight fractions and Nmers were competitors of the binding of ClRNH$^{32}$p-OdT$_{18}$, and thus of the alkylation of the protein by the radioactively labeled oligonucleotide. The value of $K_d$ for C1RNH$^{32}$P-OdT$_{18}$ for each of these proteins has previously been determined: the average $K_d$ for bFGF is 0.5 $\mu$M (Guvakova, et al., "Phosphorothioate oligodeoxynucleotides bind to basic fibroblast growth factor, inhibit its binding to cell surface receptors, and remove it from low affinity binding sites on extracellular matrix", J. Biol. Chem., 1995, (270) 2620-2627) and the average $K_d$ for laminin is 14 $\mu$M (Khaled, et al. "Multiple mechanisms may contribute to the cellular antiadhesive effects of phosphorothioate oligodeoxynucleotides", Nucl. Acids Res., 1996, (24) 737-745). In order to determine the $K_d$ for VEGF165, the concentration dependence of the modification of VEGF by C1RNH$^{32}$P-OdT$_{18}$ was examined (Fig. 3A). These results are depicted in Fig. 3B, where the concentration of modifying oligodeoxynucleotide is plotted as a function of gel band intensity. The association of VEGF with the modifying oligodeoxynucleotide exhibits approximate saturation binding and can be described by a single-site binding equation of the Michaelis-Menton type. Fig. 3C depicts the double-reciprocal replot of the data in Fig. 3B. These data are linear ($R^2$ =0.98), and the line intersects the abscissa corresponding to an apparent $K_d$ value of 33.9 $\mu$M. Similar experiments were performed for PDGF BB and HB-EGF. The $K_d$s are 4.5 and 8.7 $\mu$M, respectively.

$K_c$ was calculated from equation I as described by Cheng and Prusoff:

$$\text{Equation 1} \qquad\qquad K_c = IC_{50}/(1 + [ClRNH^{32}P\text{-}OdT_{18}]/K_d$$

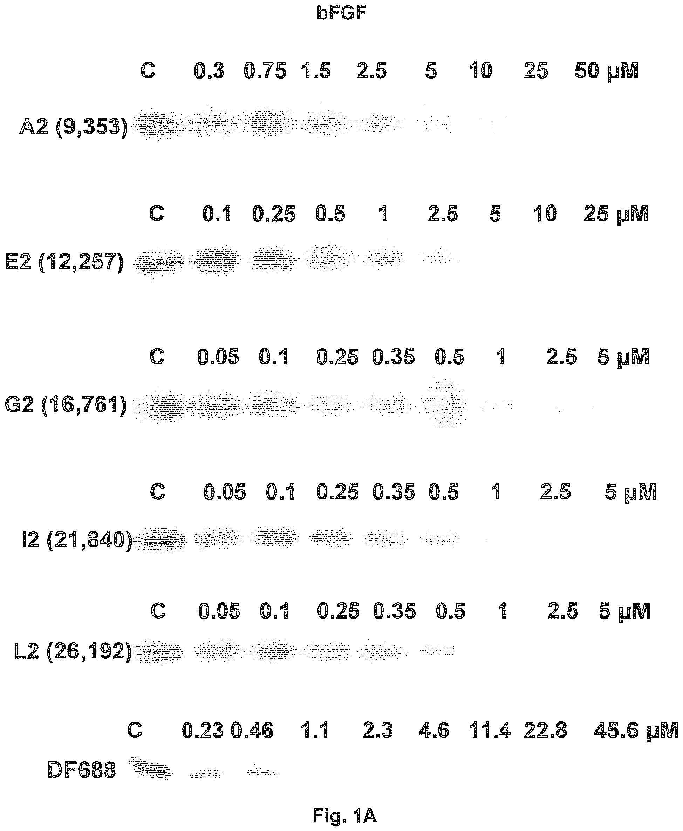

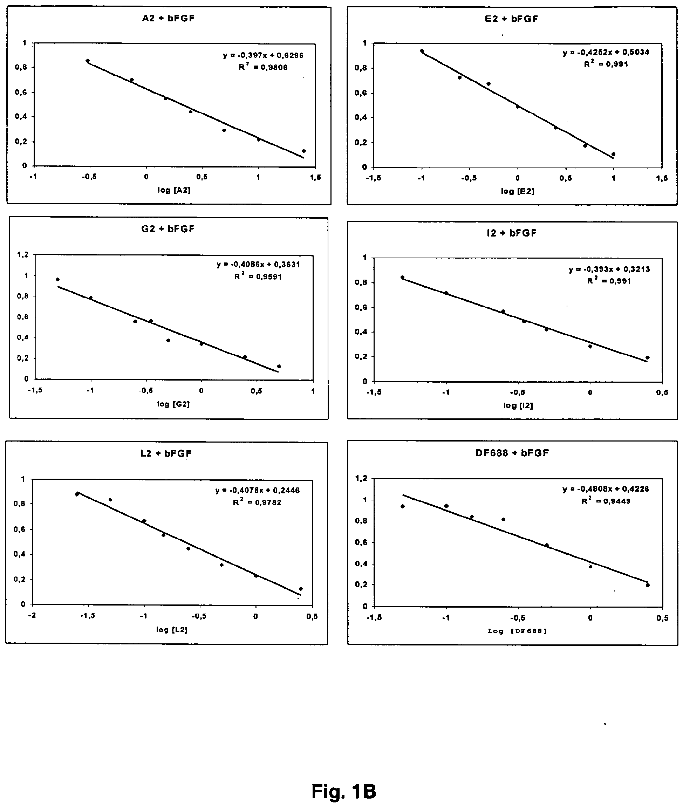

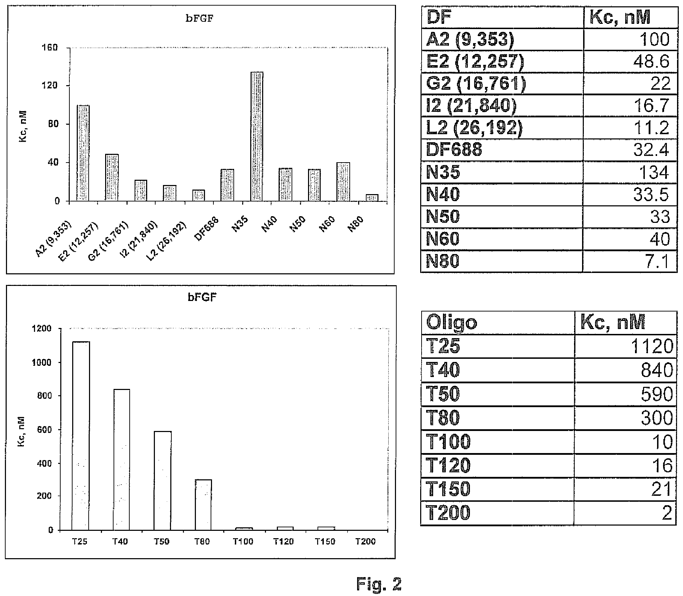

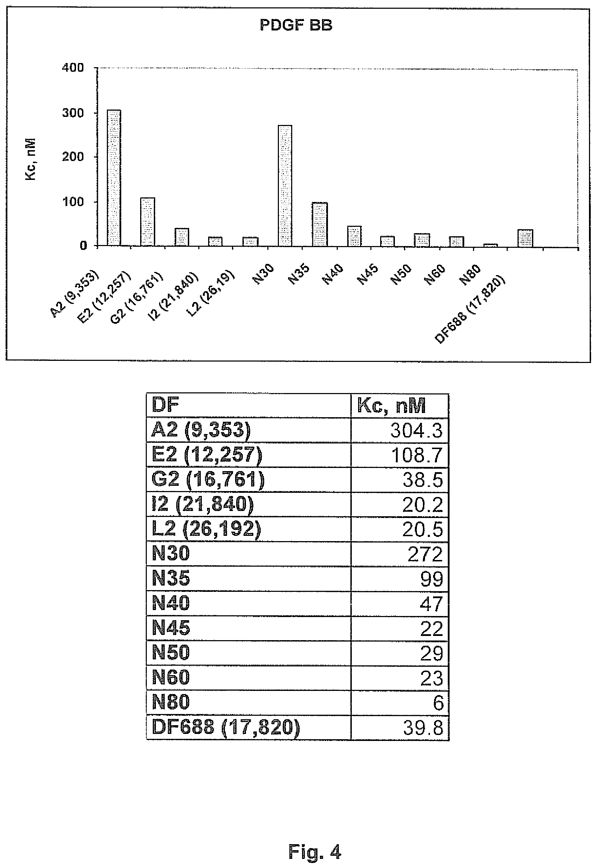

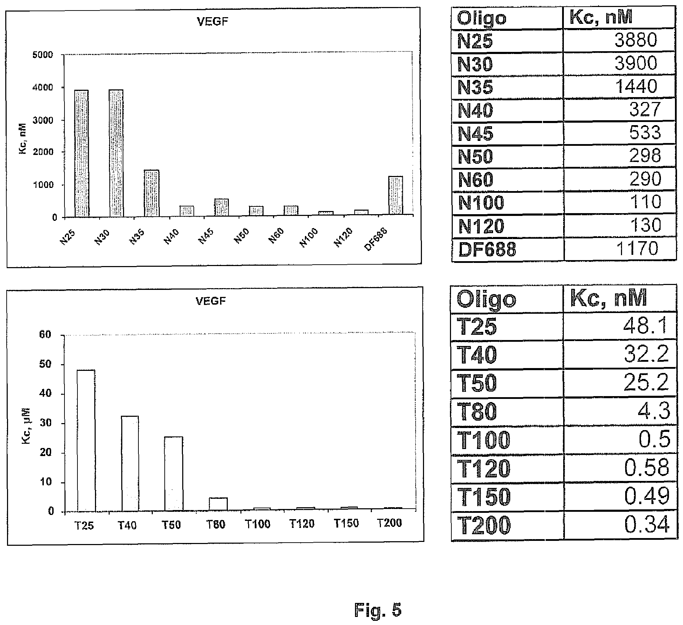

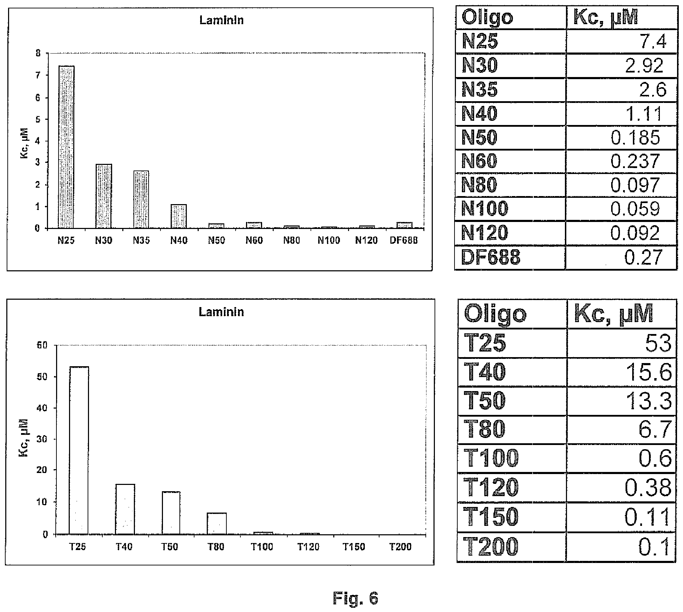

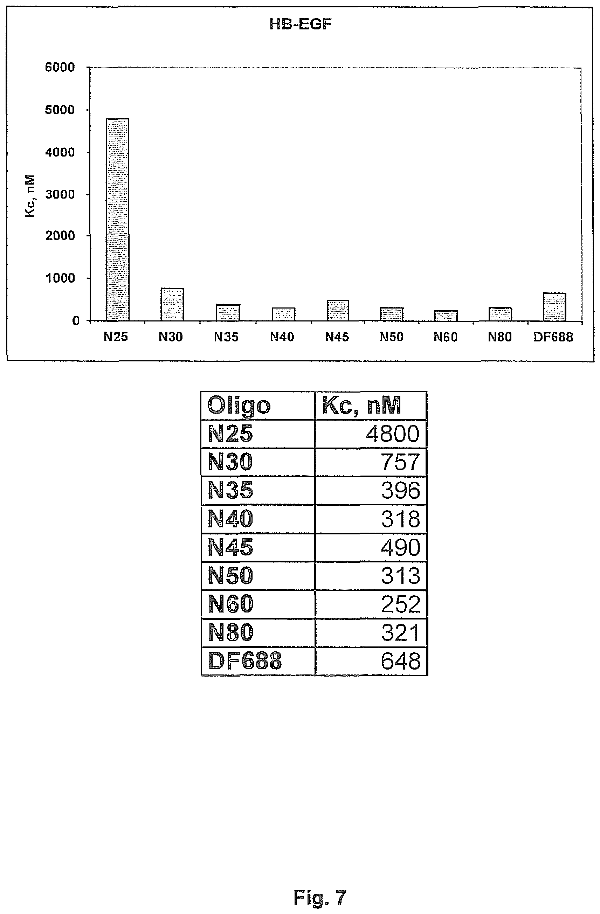

In Fig. 1A, competition for binding to bFGF is shown. As per Equation 1, a plot of the normalized intensity of the gel band versus competitor concentration was linear (Fig. 1B). The $IC_{50}$ was determined by inspection. Similar competition for binding of different competitors to all proteins of interest was also determined. The values of $K_c$, determined in an identical manner, are summarized in the Tables to Figures 2, 4, 5, 6, and 7.

*Nmers, in a length dependent fashion, and defibrotide inhibit the ability of heparin-binding growth factors to maximally stimulate the growth of SV40-transformed HMEC-1 cells an tissue culture*

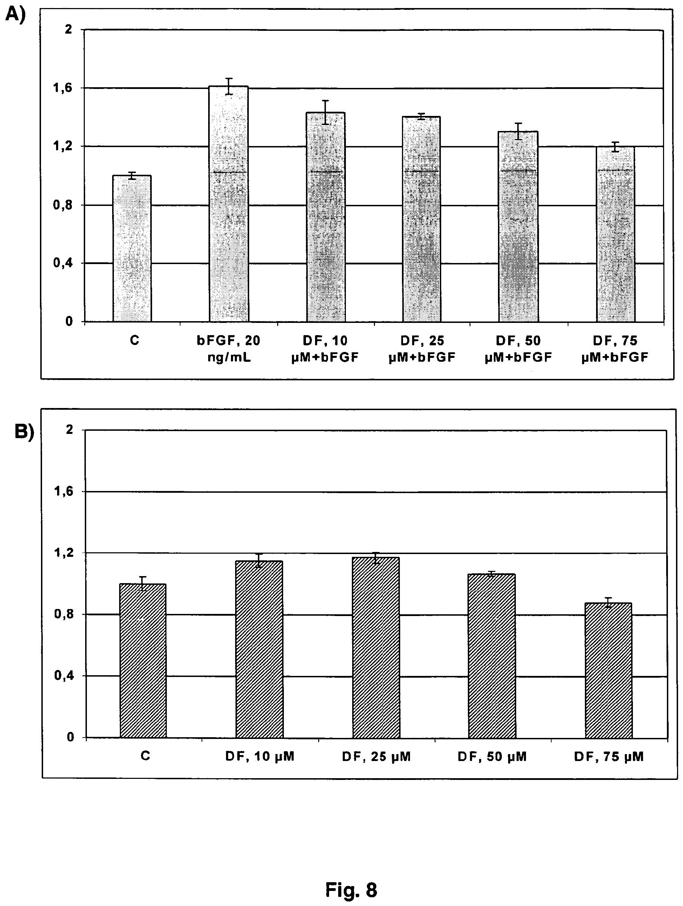

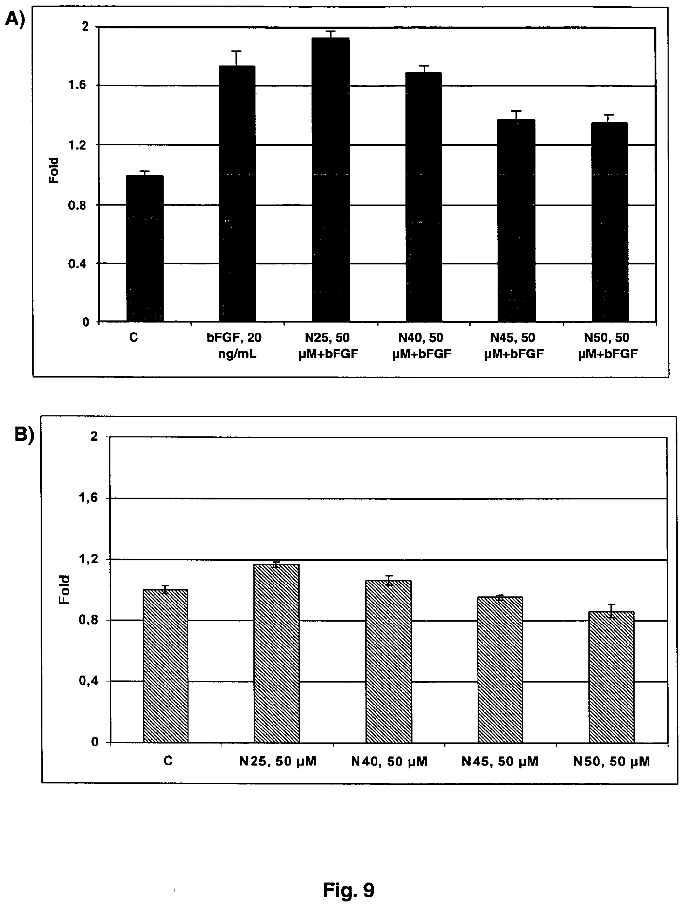

Cytokine-stimulated cell growth was determined by using sulforhodamine B (SRB). These experiments were performed in SV40-transformed HMEC-1 cells, whose growth is stimulated by bFGF. The cells were in 0% serum for 24 hours before being treated with bFGF in M199 medium containing 2.5% FBS in order to up-regulate bFGF cell surface receptors, and then incubated in medium containing 20 ng/mL bFGF with or without increasing concentrations of defibrotide or Nmers for 3 days. As shown in Fig. 8 and 9, both Nmers, in a length-dependent fashion (length of about 45 nucleotides and greater having an effect) and defibrotide cause a small (and in the case of Nmers, length-dependent), decrease in maximal bFGF-induced cell proliferation. The rate of proliferation of the HMEC-1 cells increased by 60-70% after bFGF-treatment, compared to the non-stimulated group, compared to the bFGF control. The inhibitory effect of defibrotide, when added 1 hour before bFGF, was not significantly different from that observed when added at the same time (data not shown).

*Evaluation of Defibrotide and Nmer toxicity*

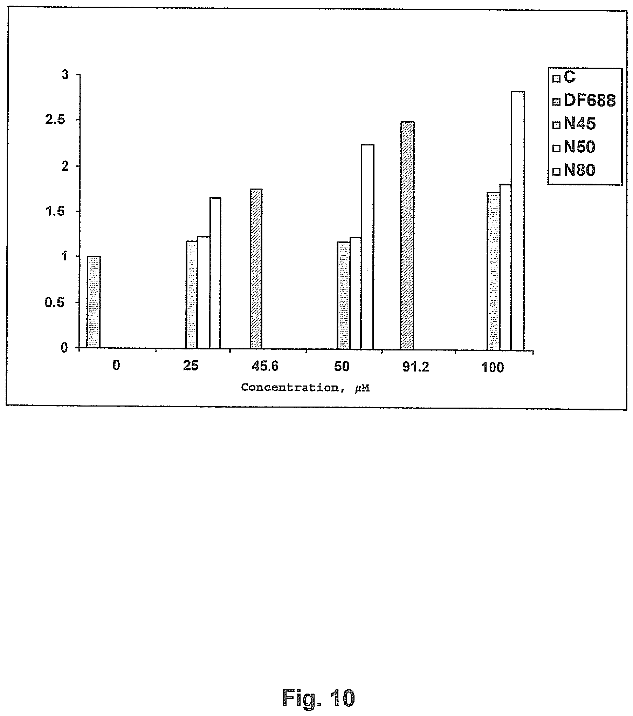

A major toxicity of defibrotide and Nmers, coagulopathy and bleeding, results from the binding of the oligonucleotides to heparin-binding members of the coagulation cascade and inhibition of their function. This anticoagulant effect was evaluated by partial thromboplastin time (PTT). Plasma from healthy volunteers was mixed with various concentrations of defibrotide or Nmers, and a standard PTT assay was performed. As shown in Fig. 10, defibrotide and Nmers do not cause significant elevation of the PTT. Only at high concentration of defibrotide, N50 or N60 ($\sim$100 $\mu$M) was there prolongation of the PTT (1.5-1.7 times compared to control) observed (Fig. 10). For a longer Nmer, N80, this effect was seen even at a 25 $\mu$M concentration.

*Defibrotide, defibrotide molecular weight fractions and Nmers increase Tissue Factor Pathway Inhibitor (TFPI) synthesis and release from HMEC-1 cells*

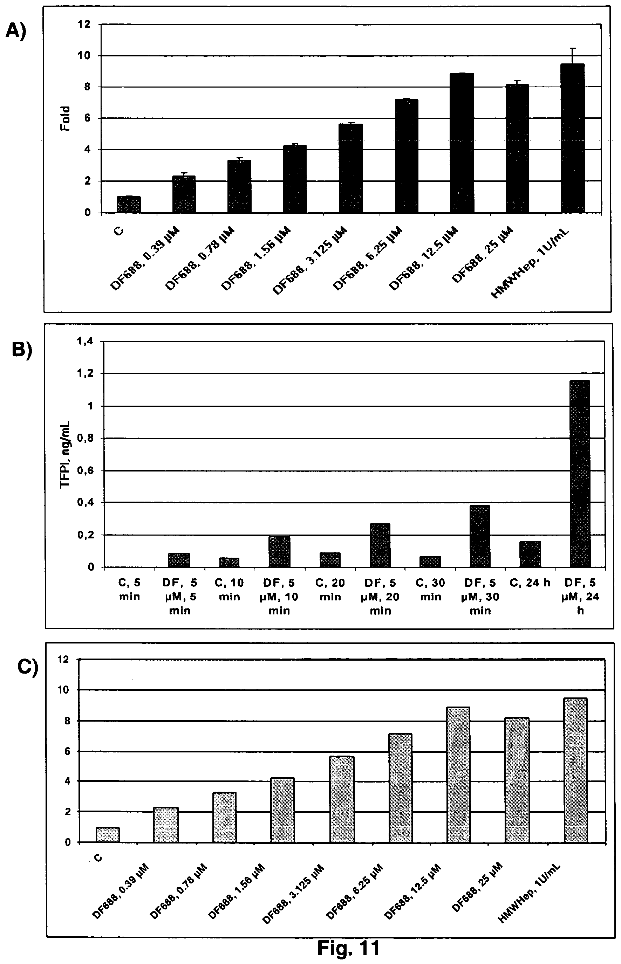

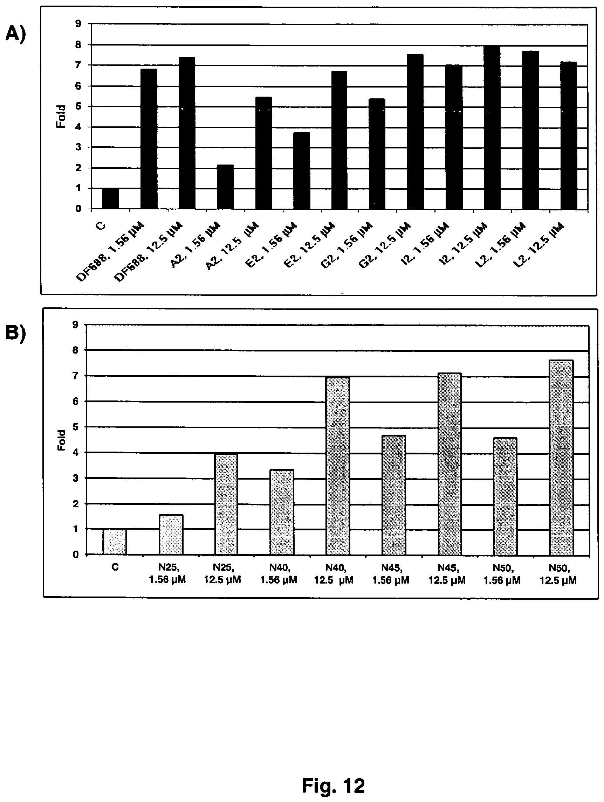

To investigate how defibrotide affects the acute and long-term release of TFPI, which is a protein that diminishes coagulopathy, from HMEC-1 cells, both concentration and time-course studies were performed. Conditioned medium from HMEC-1 cells was collected at selected time intervals, and TFPI levels determined using an ELISA assay as described by the manufacturer. As shown in Fig. 11A, 12.5 $\mu$M defibrotide caused a time-dependent increase of TFPI into the medium, with a substantial amount released after 20-30 minutes (5-6-fold increase compared to control cells). During the acute phase (30 minutes), stimulation of HMEC-1s with increasing concentrations of defibrotide caused a concentration-dependent increase of TFPI release, which plateaued at a 12.5 $\mu$M defibrotide concentration (Fig. 11C). A 24 hour-incubation of the cells with 12.5 $\mu$M defibrotide molecular weight fractions or Nmers caused a 7-8-fold increase in the TFPI in the medium compared to unstimulated cells.

*Determination of mitogenesis in C11 cells*

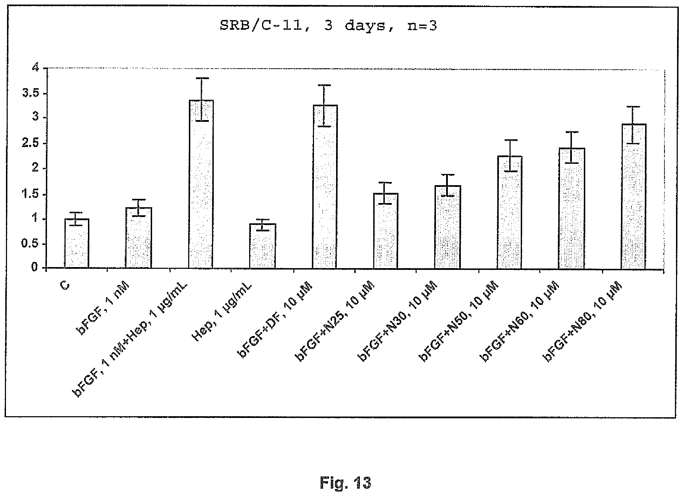

C11 clones are BAF3 mouse lymphoid cells that have been engineered to overexpress fibroblast growth factor receptor 1 (FGFR-1), to which bFGF binds with high (pM) affinity. These cells were obtained from D. Ornitz (Washington University, St. Louis). These cells have an absolute requirement for bFGF for proliferation; furthermore, it has long been known that heparin is also required for the activity of the bFGF. It had been previously demonstrated that DF and the Nmers could remove bFGF from its low affinity (nM) binding sites on extracellular matrix (Guvakova, et al., "Phosphorothioate oligodeoxynucleotides bind to basic fibroblast growth factor, inhibit its binding to cell surface receptors, and remove it from low affinity binding sites on extracellular matrix", J. Biol. Chem., 1995, (270) 2620-2627). The inventors now wanted to determine if DF and Nmers could interfere with the binding of bFGF to its high affinity binding sites. Accordingly, C11 cells were washed twice with RPMI media lacking IL-3. 2.2 x $10^4$ cells were plated per well in 48-well plates. bFGF (final 1 nM) and DF or Nmers (final 10 $\mu$M) or Heparin (1 $\mu$g/mL) were added in a total volume 200 $\mu$L. The cells (n = 3 for each experiment) were then incubated for 2-3 days, and stained with sulforhodamine blue (SRB). Cell numbers were normalized to control (proliferation in the absence of either bFGF, heparin or oligonucleotide). As can be seen in Figure 13, bFGF or heparin by themselves have little or no effect on cell proliferation after 3 days. The activity of bFGF is potentiated by both heparin and DF, demonstrating that DF can take the place of heparin. However, DF does not affect the binding of bFGF to its high-affinity binding sites. The Nmers, in a length-dependent manner, can also take the place of DF or heparin, but their activity is not quite as great as DF until a length of approximately 80mer is reached.

**CONCLUSIONS**

The synthetic phosphodiester oligonucleotides (Nmers) of the present invention can virtually recapitulate the properties of defibrotide.

Nmers and defibrotide has been evaluated and compared with respect to their abilities to bind to heparin-binding proteins (including bFGF, PDGF BB, VEGF165, laminin, and HB-EGF), and to cause TFPI release from HMEC-1 cells. The Nmers may be administered via i.v. infusion in normal saline or 5% dextrose in water to a patient afflicted with cancer or VOD (or other diseases which would be treated by administering defibrotide) at a dose of 10 mg/kg to 60 mg/kg of body weight daily in a simple dose or in divided doses for approximately 14 days. The dose may be adjusted depending on the individual patient's response to the particular course of therapy.

The values of $K_c$ for bFGF and PDGF and Nmers of various lengths (Fig. 2, 4, 5, 6, 7) demonstrate that an Nmer length approximately of at least 40 mers is sufficient for maximum Nmer activity. Such $K_c$ values also demonstrate that longer Nmers add little to the overall heparin-binding protein affinity; consequently, based both on their higher weight/dose ratio and on the difficulty to synthesize them, Nmers having a length approximately of more than 65 mers appear to be useless as an alternative to defibrotide.

The synthetic phosphodiester oligonucleotides having a length of from about 40 mers to about 65 mers may thus be used as an alternative to defibrotide and, in particular, they may be used in all the therapeutic applications disclosed above in the chapter entitled "background of the invention", which are all herein incorporated by reference in their entirety.

One of the advantages of the present invention is that the dosage of the related pharmaceutical formulations can be determined in function of the concentration of the synthetic phosphodiester oligonucleotides rather than in function of the biological activity, as it currently happens for oligonucleotide mixtures of extractive origin.

A further advantage is represented by the fact that the present invention provides for the administration of active sequences only; thus, if compared for instance to oligonucleotide mixtures of extractive origin, it provides for the administration of less oligonucleotides per dosage, with evident advantages in terms of efficacy, safety and side-effects.

Having described the present invention, it will now be apparent that many changes and modifications may be made to the above-described embodiments without departing from and the scope of the present invention.

**Claims**

1.    A pharmaceutical formulation which is an aqueous solution and which consists of: (a) water, (b) a mixture of synthetic phosphodiester oligonucleotides having a length of from 45 bases to 55 bases, in which said synthetic phosphodiester oligonucleotides have different sequences and same or different length and, optionally, (c) pharmaceutically acceptable excipients and/or adjuvants, for use in the treatment and/or prevention of veno-occlusive disease.

2.    The pharmaceutical formulation for use according to claim 1, **characterized in that** said synthetic phosphodiester oligonucleotides have different sequences and same length.

3.    The pharmaceutical formulation for use according to claim l, **characterized in that** said oligonucleotides have a length of from about 50 bases to about 55 bases.

4.    The pharmaceutical formulation for use according to claim 1, **characterized in that** said oligonucleotides are single stranded.

5.    The pharmaceutical formulation for use according to claim 1, **characterized in that** the sequences of said oligonucleotides are DNA and/or RNA sequences.

6.    The pharmaceutical formulation for use according to claim 1, **characterized in that** the sequences of said oligonucleotides are random sequences.

7.    The pharmaceutical formulation for use according to claim 1, **characterized in that** the purine bases of said oligonucleotides are selected from guanine, adenine, xanthine and hypoxanthine and the pyrimidine bases are selected from cytosine, thymine, methylcytosine and uracil.

8.    The pharmaceutical formulation for use according to claim 1, **characterized in that** the sugar of said oligonucleotides is selected from ribose and deoxyribose.

9. The pharmaceutical formulation for use according to any of the previous claims, **characterized in that** it is to be administered intravenously.

10. The pharmaceutical formulation for use according to any of the previous claims, **characterized in that** it is to be administered to a mammalian.

11. The pharmaceutical formulation for use according to claim 10, **characterized in that** said mammalian is a human.

12. The pharmaceutical formulation for use according to claim 1, **characterized by** having an oligonucleotide concentration of from 5 to 60 micromoles/liter.

13. The pharmaceutical Formulation for use according to claim 12, **characterized by** having an oligonucleotide concentration of from 10 to 50 micromoles/liter.

**Patentansprüche**

1. Pharmazeutische Formulierung, welche eine wässrige Lösung ist, und welche besteht aus:

   (a) Wasser,
   (b) eine Mischung synthetischer Phosphodiesteroligonukleotide mit einer Länge von 45 Basen bis 55 Basen, in welcher diese synthetischen Phosphodiesteroligonukleotide verschiedene Sequenzen haben und gleiche oder verschiedene Länge haben, und, optional,
   (c) pharmazeutisch akzeptable Exipients und/oder Hilfsstoffe zur Verwendung in der Behandlung und/oder Prävention von venöser Verschlusskrankheit.

2. Pharmazeutische Formulierung zur Verwendung gemäß Anspruch 1, **dadurch gekennzeichnet, dass** die synthetischen Phosphodiesteroligonukleotide verschiedene Sequenzen und gleiche Länge haben.

3. Pharmazeutische Formulierung zur Verwendung gemäß Anspruch 1, **dadurch gekennzeichnet, dass** die Oligonukleotide eine Länge von ungefähr 50 Basen bis ungefähr 55 Basen haben.

4. Pharmazeutische Formulierung zur Verwendung gemäß Anspruch 1, **dadurch gekennzeichnet, dass** die Oligonukleotide einzelsträngig sind.

5. Pharmazeutische Zusammensetzung zur Verwendung gemäß Anspruch 1, **dadurch gekennzeichnet, dass** die Sequenzen der Oligonukleotide DNA- und/oder RNA-Sequenzen sind.

6. Pharmazeutische Formulierung zur Verwendung gemäß Anspruch 1, **dadurch gekennzeichnet, dass** die Sequenzen der Oligonukleotide Zufallssequenzen sind.

7. Pharmazeutische Formulierung zur Verwendung gemäß Anspruch 1, **dadurch gekennzeichnet, dass** die Purinbasen der Oligonukleotide ausgewählt sind aus Guanin, Adenin, Xanthin und Hypoxanthin und die Pyrimidinbasen ausgewählt sind aus Cytosin, Thymin, Methylcytosin und Uracil.

8. Pharmazeutische Formulierung zur Verwendung gemäß Anspruch 1, **dadurch gekennzeichnet, dass** der Zucker der Oligonukleotide ausgewählt ist aus Ribose und Deoxyribose.

9. Pharmazeutische Formulierung zur Verwendung gemäß irgendeinem der vorhergehenden Ansprüche, **dadurch gekennzeichnet, dass** sie intravenös verabreicht wird.

10. Pharmazeutische Formulierung zur Verwendung gemäß irgendeinem der vorhergehenden Ansprüche, **dadurch gekennzeichnet, dass** sie einem Säuger verabreicht wird.

11. Pharmazeutische Formulierung zur Verwendung gemäß Anspruch 10, **dadurch gekennzeichnet, dass** der Säuger ein Mensch ist.

12. Pharmazeutische Formulierung zur Verwendung gemäß Anspruch 1, **dadurch gekennzeichnet, dass** sie eine Oligonukleotid-Konzentration von 5 bis 60 mmol/l hat.

13. Pharmazeutische Formulierung zur Verwendung gemäß Anspruch 12, **dadurch gekennzeichnet, dass** sie eine Oligonukleotid-Konzentration von 10-50 µmol/l hat.

## Revendications

1. Formulation pharmaceutique qui est une solution aqueuse et qui consiste en : (a) de l'eau, (b) un mélange d'oligonucléotides phosphodiesters synthétiques ayant une longueur de 45 bases à 55 bases, dans laquelle lesdits oligonucléotides phosphodiesters synthétiques possèdent des séquences différentes et une longueur identique ou différente et, facultativement, (c) des excipients et/ou adjuvants pharmaceutiquement acceptables, pour une utilisation dans le traitement et/ou la prévention de la maladie veino-occlusive.

2. Formulation pharmaceutique destinée à être utilisée selon la revendication 1, **caractérisée en ce que** lesdits oligonucléotides phosphodiesters synthétiques possèdent des séquences différentes et une longueur identique.

3. Formulation pharmaceutique destinée à être utilisée selon la revendication 1, **caractérisée en ce que** lesdits oligonucléotides ont une longueur d'environ 50 bases à environ 55 bases.

4. Formulation pharmaceutique destinée à être utilisée selon la revendication 1, **caractérisée en ce que** lesdits oligonucléotides sont simple brin.

5. Formulation pharmaceutique destinée à être utilisée selon la revendication 1, **caractérisée en ce que** les séquences desdits oligonucléotides sont des séquences d'ADN et/ou d'ARN.

6. Formulation pharmaceutique destinée à être utilisée selon la revendication 1, **caractérisée en ce que** les séquences desdits oligonucléotides sont des séquences aléatoires.

7. Formulation pharmaceutique destinée à être utilisée selon la revendication 1, **caractérisée en ce que** les bases purine desdits oligonucléotides sont sélectionnées parmi la guanine, l'adénine, la xanthine et l'hypoxanthine et les bases pyrimidine sont sélectionnés parmi la cytosine, la thymine, la méthylcytosine et l'uracile.

8. Formulation pharmaceutique destinée à être utilisée selon la revendication 1, **caractérisée en ce que** le sucre desdits oligonucléotides est sélectionné parmi le ribose et le désoxyribose.

9. Formulation pharmaceutique destinée à être utilisée selon l'une quelconque des revendications précédentes, **caractérisée en ce qu'**elle doit être administrée par voie intraveineuse.

10. Formulation pharmaceutique destinée à être utilisée selon l'une quelconque des revendications précédentes, **caractérisée en ce qu'**elle doit être administrée à un mammifère.

11. Formulation pharmaceutique destinée à être utilisée selon la revendication 10, **caractérisée en ce que** ledit mammifère est un humain.

12. Formulation pharmaceutique destinée à être utilisée selon la revendication 1, **caractérisée en ce qu'**elle possède une concentration en oligonucléotides comprise entre 5 et 60 micromoles/litre.

13. Formulation pharmaceutique destinée à être utilisée selon la revendication 12, **caractérisée en ce qu'**elle possède une concentration en oligonucléotides comprise entre 10 et 50 micromoles/litre.

bFGF

| | C | 0.3 | 0.75 | 1.5 | 2.5 | 5 | 10 | 25 | 50 µM |
|---|---|---|---|---|---|---|---|---|---|
| A2 (9,353) | | | | | | | | | |

| | C | 0.1 | 0.25 | 0.5 | 1 | 2.5 | 5 | 10 | 25 µM |
|---|---|---|---|---|---|---|---|---|---|
| E2 (12,257) | | | | | | | | | |

| | C | 0.05 | 0.1 | 0.25 | 0.35 | 0.5 | 1 | 2.5 | 5 µM |
|---|---|---|---|---|---|---|---|---|---|
| G2 (16,761) | | | | | | | | | |

| | C | 0.05 | 0.1 | 0.25 | 0.35 | 0.5 | 1 | 2.5 | 5 µM |
|---|---|---|---|---|---|---|---|---|---|
| I2 (21,840) | | | | | | | | | |

| | C | 0.05 | 0.1 | 0.25 | 0.35 | 0.5 | 1 | 2.5 | 5 µM |
|---|---|---|---|---|---|---|---|---|---|
| L2 (26,192) | | | | | | | | | |

| | C | 0.23 | 0.46 | 1.1 | 2.3 | 4.6 | 11.4 | 22.8 | 45.6 µM |
|---|---|---|---|---|---|---|---|---|---|
| DF688 | | | | | | | | | |

Fig. 1A

**Fig. 1B**

13

| DF | Kc, nM |
|---|---|
| A2 (9,353) | 100 |
| E2 (12,257) | 48.6 |
| G2 (16,761) | 22 |
| I2 (21,840) | 16.7 |
| L2 (26,192) | 11.2 |
| DF688 | 32.4 |
| N35 | 134 |
| N40 | 33.5 |
| N50 | 33 |
| N60 | 40 |
| N80 | 7.1 |

| Oligo | Kc, nM |
|---|---|
| T25 | 1120 |
| T40 | 840 |
| T50 | 590 |
| T80 | 300 |
| T100 | 10 |
| T120 | 16 |
| T150 | 21 |
| T200 | 2 |

Fig. 2

**A)**  0 .1   0.25   0.5   1   2.5   5   10   25   50   100 µM

**B)**

PDGF BB

**C)**

PDGF BB

$y = 0{,}0431x + 0{,}0096$
$R^2 = 0{,}9934$

**Fig. 3**

PDGF BB

| DF | Kc, nM |
|---|---|
| A2 (9,353) | 304.3 |
| E2 (12,257) | 108.7 |
| G2 (16,761) | 38.5 |
| I2 (21,840) | 20.2 |
| L2 (26,192) | 20.5 |
| N30 | 272 |
| N35 | 99 |
| N40 | 47 |
| N45 | 22 |
| N50 | 29 |
| N60 | 23 |
| N80 | 6 |
| DF688 (17,820) | 39.8 |

Fig. 4

| Oligo | Kc, nM |
|-------|--------|
| N25 | 3880 |
| N30 | 3900 |
| N35 | 1440 |
| N40 | 327 |
| N45 | 533 |
| N50 | 298 |
| N60 | 290 |
| N100 | 110 |
| N120 | 130 |
| DF688 | 1170 |

| Oligo | Kc, nM |
|-------|--------|
| T25 | 48.1 |
| T40 | 32.2 |
| T50 | 25.2 |
| T80 | 4.3 |
| T100 | 0.5 |
| T120 | 0.58 |
| T150 | 0.49 |
| T200 | 0.34 |

Fig. 5

Laminin

| Oligo | Kc, µM |
|-------|--------|
| N25   | 7.4    |
| N30   | 2.92   |
| N35   | 2.6    |
| N40   | 1.11   |
| N50   | 0.185  |
| N60   | 0.237  |
| N80   | 0.097  |
| N100  | 0.059  |
| N120  | 0.092  |
| DF688 | 0.27   |

Laminin

| Oligo | Kc, µM |
|-------|--------|
| T25   | 53     |
| T40   | 15.6   |
| T50   | 13.3   |
| T80   | 6.7    |
| T100  | 0.6    |
| T120  | 0.38   |
| T150  | 0.11   |
| T200  | 0.1    |

Fig. 6

18

HB-EGF

| Oligo | Kc, nM |
|-------|--------|
| N25 | 4800 |
| N30 | 757 |
| N35 | 396 |
| N40 | 318 |
| N45 | 490 |
| N50 | 313 |
| N60 | 252 |
| N80 | 321 |
| DF688 | 648 |

Fig. 7

**Fig. 8**

Fig. 9

Fig. 10

Fig. 11

**Fig. 12**

Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3770720 A
- US 3899481 A
- US 3829567 A
- US 4694134 A
- US 4693995 A
- US 5081109 A
- US 5116617 A
- US 5977083 A
- US 6046172 A
- US 6699985 B
- US 5624912 A
- US 7338777 B
- EP 1276497 A
- WO 2005023273 A
- WO 2006094916 A
- US 4985552 A
- US 5223609 A
- EP 1325162 A

### Non-patent literature cited in the description

- **KORNBLUM et al.** *Oligonucleotides,* 2006, vol. 16, 105-114
- **SAMBROOK et al.** Molecular Cloning: A Laboratory Manual. 1989
- DNA Cloning: A Practical Approach
- Oligonucleotide Synthesis. 1984
- Nucleic Acid Hybridization. 1985
- **PERBAL.** A Practical Guide to Molecular Cloning. 1984
- **AUSUBEL et al.** Current protocols in Molecular Biology. John Wiley and Sons, 1987
- **BONIFACINO et al.** Current Protocols in Cell Biology. John Wiley & Sons, 1999
- **YAKUBOV et al.** Oligonucleotides interact with recombinant CD4 at multiple sites. *J. Biol. Chem.,* 1993, vol. 268, 19918-18823
- **GUVAKOVA et al.** Phosphorothioate oligodeoxynucleotides bind to basic fibroblast growth factor, inhibit its binding to cell surface receptors, and remove it from low affinity binding sites on extracellular matrix. *J. Biol. Chem.,* 1995, vol. 270, 2620-2627
- **KHALED et al.** Multiple mechanisms may contribute to the cellular antiadhesive effects of phosphorothioate oligodeoxynucleotides. *Nucl. Acids Res.,* 1996, vol. 24, 737-745